# EUROPÄISCHE PATENTANMELDUNG

(11) **EP 3 415 609 A1**
(43) Veröffentlichungstag der Anmeldung: **19.12.2018**
(21) Anmeldenummer: 18176286.5
(22) Anmeldetag: 06.06.2018
(51) Int. Cl.: C12M 1/107, C12M 1/00, H01M 8/0656, H01M 8/04082, C01B 3/00, H01M 16/00

(54) **VERFAHREN ZUM SPEICHERN UND LAGERN VON WASSERSTOFF**

(30) Priorität: 13.06.2017 DE 102017113027
(71) Anmelder: N-ERGIE Aktiengesellschaft, 90429 Nümberg (DE)
(72) Erfinder: Fleischer, Dr. Patrick, 91207 Lauf an der Pegnitz (DE)
(74) Vertreter: Ellwanger, Arndt

(57) **Zusammenfassung**

Die vorliegende Erfindung betrifft ein Verfahren zum Speichern und Lagern von Wasserstoff, umfassend zwei Verfahrensschritte. Im ersten Verfahrensschritt wird der Wasserstoff gespeichert, indem Wasserstoff mit einem Träger (T1) in Kontakt gebracht wird, wodurch ein mit Wasserstoff beladener Träger (T2) erhalten wird. Der Wasserstoff wird dabei vorzugsweise chemisch, insbesondere im Rahmen einer Hydrierung, an den Träger (T1) gebunden. Im zweiten Verfahrensschritt erfolgt die Lagerung des mit Wasserstoff beladenen Träger (T2) in einer Vorrichtung (V1), die Bestandteil einer Biogasanlage ist oder war. Der im Träger (T2) gespeicherte Wasserstoff kann anschließend aus dem Träger (T2) wieder freigesetzt und nachfolgend zur Erzeugung von Strom und gegebenenfalls Wärme verwendet werden. Das erfindungsgemäße Verfahren kann in eine bestehende Biogasanlage integriert oder eine Biogasanlage kann entsprechend umgerüstet werden. Weiterhin betrifft die vorliegende Erfindung ein Verfahren zur Erzeugung von elektrischem Strom und gegebenenfalls von Wärme, wobei zunächst das Verfahren zum Speichern und Lagern von Wasserstoff durchgeführt wird.

## Beschreibung

Die vorliegende Erfindung betrifft ein Verfahren zum Speichern und Lagern von Wasserstoff, umfassend zwei Verfahrensschritte. Im ersten Verfahrensschritt wird der Wasserstoff gespeichert, indem Wasserstoff mit einem Träger (T1) in Kontakt gebracht wird, wodurch ein mit Wasserstoff beladener Träger (T2) erhalten wird. Der Wasserstoff wird dabei vorzugsweise chemisch, insbesondere im Rahmen einer Hydrierung, an den Träger (T1) gebunden. Im zweiten Verfahrensschritt erfolgt die Lagerung des mit Wasserstoff beladenen Träger (T2) in einer Vorrichtung (V1), die Bestandteil einer Biogasanlage ist oder war. Der im Träger (T2) gespeicherte Wasserstoff kann anschließend aus dem Träger (T2) wieder freigesetzt und nachfolgend zur Erzeugung von Strom und gegebenenfalls Wärme verwendet werden. Das erfindungsgemäße Verfahren kann in eine bestehende Biogasanlage integriert oder eine Biogasanlage kann entsprechend umgerüstet werden. Weiterhin betrifft die vorliegende Erfindung ein Verfahren zur Erzeugung von elektrischem Strom und gegebenenfalls von Wärme, wobei zunächst das Verfahren zum Speichern und Lagern von Wasserstoff durchgeführt wird.

Biogasanlagen als solche sind bereits seit langem bekannt (siehe beispielsweise die freie Enzyklopädie Wikipedia unter dem Begriff "Biogasanlage"; https://de.wikipedia.org/wiki/biogasanlage; Version vom 19. April 2017). Eine Biogasanlage dient zur Erzeugung von Biogas durch Vergärung von Biomasse. Biogasanlagen werden in der Regel in landwirtschaftlichen Betrieben eingesetzt, wo beispielsweise tierische Exkremente (Gülle/Festmist) oder Energiepflanzen als Substrat eingesetzt werden. Als Nebenprodukt wird häufig ein als Gärrest bezeichneter Dünger produziert. Das bei der Biogasanlage als Hauptprodukt anfallende Biogas wird häufig zur dezentral gekoppelten Strom- und Wärmeerzeugung (Kraft-Wärme-Kopplung) in Blockheizkraftwerken genutzt. Dabei wird das Biogas, das gegebenenfalls noch getrocknet und/oder entschwefelt wird, einem Gasmotor zugeführt, der das Biogas in elektrischen Strom sowie gegebenenfalls in Wärme verwandelt. Der so produzierte Strom wird in das Netz eingespeist. Meistens erfolgt die Herstellung des elektrischen Stroms aus dem Biogas direkt vor Ort als Bestandteil einer Biogasanlage. Alternativ ist es auch möglich, dass das in einer Biogasanlage erzeugte Biogas auf Erdgasqualität gereinigt und als Biomethan (Bioerdgas) in das Erdgasnetz eingespeist wird.

Zentraler Bestandteil einer solchen Biogasanlage ist in der Regel ein Biogasreaktor, der auch als Fermenter bezeichnet wird (siehe auch Wikipedia; https://de.wikipedia.org/wiki/bioreaktor; Version vom 21. April 2017). In diesem Fermenter wird in Abhängigkeit von der eingebrachten Biomasse durch spezifische biologische Prozesse (Biokonversion, Biokatalyse) das Biogas erzeugt. In der Praxis gibt es zahlreiche Hersteller von solchen Reaktoren/Fermentern, die sich hinsichtlich Größe und Ausstattung sowie Art und Dimension der Zuleitungen der im Fermenter enthaltenen Einbauten, wie Rührwerke oder etwaige vorhandene Heizsysteme im Inneren des Fermenters, unterscheiden. Eine Biogasanlage kann auch zwei oder mehrere Fermenter enthalten. Weitere Fermenter werden auch als Nachgärer bezeichnet/eingesetzt. Weiterhin verfügen Biogasanlagen häufig über zahlreiche Vorrats- oder Lagerbehälter, um beispielsweise die zu vergärende Biomasse oder den Gärrückstand zu lagern. Die einzelnen Vorrichtungselemente einer Bioanlage sind in der Regel durch ein entsprechendes Leitungssystem sowie durch Pumpen miteinander verbunden.

DE-A 10 2014 010 641 betrifft einen Fermenter zur Erzeugung von Biogas durch anaerobe Fermentation mit mindestens einer Fermentierkammer und mit mindestens einer Zuführeinrichtung, wobei die Zuführeinrichtung zum Eintragen von Fermentiergut in die Fermentierkammer ausgebildet ist. Die Fermenter können darüber hinaus ein Rührwerkzeug aufweisen. Weiterhin wird eine Anlage zur Erzeugung von Biogas mit mindestens einem Fermenter sowie ein Verfahren zur Erzeugung von Biogas offenbart. Die Anlage zur Erzeugung von Biogas kann in ein Blockheizkraftwerk mit einem Verbrennungsmotor integriert werden.

Ein wesentliches Problem von Biogasanlagen ist deren wirtschaftliche Betreibung. Zumindest in Deutschland, aber auch in anderen europäischen Ländern sinngemäß, wird der Betrieb von Biogasanlagen im Rahmen der erneuerbaren Energien wirtschaftlich gefördert. Allerdings sind diese staatlichen Förderungen im Zusammenhang mit der Erzeugung von erneuerbaren Energien nicht unbegrenzt erhältlich, sondern werden in absehbarer Zeit, insbesondere im Zusammenhang mit dem Betrieb von Biogasanlagen, reduziert oder sogar auslaufen. Ohne staatliche Hilfe ist es für viele landwirtschaftliche Betriebe schwierig, insbesondere kleinere Biogasanlagen weiterhin wirtschaftlich zu betreiben. Ein weiteres Problem beim Betreiben von Biogasanlagen ist in der Gefahr von Gasleakagen bzw. dem Wärmeverlust zu sehen. Um das in der Regel brennbare Biogas zu handhaben bzw. zu lagern sind erhöhte Sicherheitsvorkehrungen notwendig.

DE-A 10 2012 005 023 offenbart eine Anordnung sowie ein Verfahren zur autonomen Bereitstellung von Elektrizität über Wasserstoff. Hierbei wird zunächst elektrischer Strom aus einer regenerativen Quelle bereitgestellt, der zur Herstellung von Wasserstoff aus Wasser in mindestens einem Elektrolyseur verwendet wird. Der so hergestellte Wasserstoff wird in einen ersten chemischen Reaktor überführt, der mindestens ein Substrat mit einem ausgedehnten pi-konjugierten System enthält, wobei in diesem ersten chemischen Reaktor eine zumindest teilweise Hydrierung des Substrats durchgeführt wird. Anschließend wird dieses zumindest teilweise hydrierte Substrat in einen Speichertank überführt. Aus diesem Speichertank wird das zumindest teilweise hydrierte Substrat wiederum in einen zweiten chemischen Reaktor überführt, wo eine Dehydrierung dieses Substrates unter Freisetzung von Wasserstoff erfolgt. Dieser im zweiten chemischen Reaktor erzeugte Wasserstoff wird in eine Anlage oder Maschine zur Wandlung des Wasserstoffs in elektrische Energie, vorzugsweise in eine Brennstoffzelle, überführt. Von dort wird elektrischer Strom an den Ort des Bedarfs abgegeben.

Das in DE-A 10 2012 005 023 beschriebene Verfahren bzw. die entsprechende Anlage eignet sich beispielsweise als Notstromaggregat oder an Orten, an denen ein öffentliches Stromnetz fehlt, sowie in Krisensituationen, wie Kriegen. Anwendungsgebiete können Krankenhäuser, chemische Anlagen, Computerzentralen oder auch Kernkraftwerke sein. Letztendlich wird bei diesem Verfahren Wasserstoff zwischengespeichert, was gegebenenfalls auch über einen längeren Zeitraum erfolgen kann, um ihn im Bedarfsfall zur Stromerzeugung einzusetzen. Der anfänglich bereitgestellte elektrische Strom, der aus regenerativen Quellen stammt, mit dem der Wasserstoff erzeugt wird, kann beispielsweise aus Wasserkraft, Windenergie, solarer Strahlung oder auch aus Biogas stammen. Die Durchführung des Verfahrens im Rahmen einer Biogasanlage ist in DE-A 10 2012 005 023 jedoch nicht offenbart.

Ein sinngemäßes Verfahren bzw. eine entsprechende Anordnung ist in DE-A 10 2011 121 704 offenbart. Die Anordnung (Vorrichtung) wird insbesondere zur Pufferung von Überschuss-Elektrizität in Gebäuden eingesetzt. Das entsprechende Verfahren dient zur Energiespeicherung in Gebäuden. Das Verfahren sowie die entsprechende Anordnung erfordern zwingend die Verwendung einer Brennstoffzelle zur Oxidation des in dem zweiten chemischen Reaktor freigesetzten Wasserstoffs unter Freisetzung von Energie.

DE-A 10 2014 223 427 offenbart ein Verfahren sowie die entsprechende Anlage zum Erzeugen und Speichern von Wasserstoff. In dem Verfahren wird zunächst ein Rohstoffgasgemisch bereitgestellt, das mindestens eine Kohlenwasserstoffverbindung aufweist. Dieses Rohstoffgasgemisch wird vorbehandelt, um ein Zwischengasgemisch mit erhöhtem Wasserstoffanteil herzustellen. Anschließend erfolgt ein Kontaktieren des Zwischengasgemisches mit einen Wasserstoff-Trägermedium zum Hydrieren des Wasserstoff-Trägermediums.

DE-B 10 2013 223 589 betrifft eine Anlage sowie ein entsprechendes Verfahren zum Speichern von Energie. Die Anlage umfasst eine Stromerzeugungseinheit, eine Wasserstofferzeugungseinheit, eine Wasserstoffspeicherungsvorrichtung, eine Wärmeerzeugungseinheit sowie eine Wärmespeicherungseinheit. Die Wasserstoffspeicherungsvorrichtung umfasst wiederum eine Beladeeinheit zum Beladen eines Trägermediums mit dem in der Wasserstofferzeugungseinheit erzeugten Wasserstoff sowie eine Entladeeinheit zum Entladen des Wasserstoffs von dem beladenen Trägermedium. Die Wärmespeicherungseinheit ist mit der Entladeeinheit zum Bereitstellen von Wärme verbunden. Weiterhin ist die Stromerzeugungseinheit mit der Wärmeerzeugungseinheit zur Bereitstellung von elektrischem Strom für die Wärmeerzeugung verbunden.

Die der vorliegenden Erfindung zugrunde liegende Aufgabe besteht in der Bereitstellung eines neuen Verfahrens zum Speichern von Energie, insbesondere von Wasserstoff, und/oder in einem Verfahren zur Erzeugung von Energie, insbesondere von elektrischem Strom sowie gegebenenfalls von Wärme.

Gelöst wird die Aufgabe durch ein Verfahren zum Speichern und Lagern von Wasserstoff umfassend die nachfolgenden Schritte a) und b):
a) Inkontaktbringen eines Trägers (T1) mit Wasserstoff zur Speicherung des Wasserstoffs unter Erhalt eines mit Wasserstoff beladenen Trägers (T2),
b) Lagerung des mit Wasserstoff beladenen Trägers (T2) in einer Vorrichtung (V1), die Bestandteil einer Biogasanlage ist oder war.

Weiterhin wird die Aufgabe gelöst durch ein Verfahren zur Erzeugung von elektrischem Strom und gegebenenfalls von Wärme umfassend die nachfolgenden Schritte a) bis e), wobei das Verfahren in der Reihenfolge der Schritte c), a), b), d) und e) durchgeführt wird:
c) Erzeugung von Wasserstoff in einem Elektrolyseur unter Verwendung von Wasser und elektrischem Strom,
a) Inkontaktbringen eines Trägers (T1) mit dem in Schritt c) erzeugten Wasserstoff zur Speicherung des Wasserstoffs unter Erhalt eines mit Wasserstoff beladenen Trägers (T2),
b) Lagerung des mit Wasserstoff beladenen Trägers (T2) in einer Vorrichtung (V1), die Bestandteil einer Biogasanlage ist oder war,
d) Freisetzung von Wasserstoff aus dem mit Wasserstoff beladenen Träger (T2) im Anschluss an die Lagerung gemäß Schritt b),
e) Überführung des in Schritt d) erhaltenen Wasserstoffs in eine Vorrichtung (V2), wobei in der Vorrichtung (V2) Wasserstoff in elektrischen Strom und gegebenenfalls in Wärme umgewandelt wird.

Ein wesentlicher Vorteil des erfindungsgemäßen Verfahrens ist darin begründet, dass die jeweilige Infrastruktur der in Deutschland bzw. Europa zahlreich vorhandenen Biogasanlagen verwendet werden kann, um das erfindungsgemäße Verfahren durchzuführen. Dadurch können die Anfangsinvestitionskosten gegenüber einem kompletten Neubau/dem Kauf einer kompletten neuen Anlage deutlich reduziert werden. Wesentliche Vorrichtungskomponenten jeder gängigen Biogasanlage können für das erfindungsgemäße Verfahren verwendet werden, ohne dass dabei Kosten im großen Umfang im Zusammenhang mit der Umrüstung einzelner Vorrichtungselemente einer Biogasanlage anfallen müssen. Weiterhin können Vorrichtungselemente, die entweder direkt zur Biogasanlage gehören oder ihr unmittelbar angeschlossen sind, wie ein Blockheizkraftwerk oder ein darin enthaltener Gasmotor, auch ohne weitere Umrüstung direkt im erfindungsgemäßen Verfahren sinngemäß zum Betrieb bei einer Biogasanlage mit nachfolgender Stromerzeugung verwendet werden.

Ebenfalls ist es als vorteilhaft anzusehen, dass das erfindungsgemäße Verfahren auch parallel und/oder zeitversetzt zum herkömmlichen Betrieb einer Biogasanlage durchgeführt werden kann. So ist es denkbar, dass zusätzlich zum bisherigen "konventionellen Betrieb" einer Biogasanlage entsprechende Vorrichtungselemente ergänzt werden, um das erfindungsgemäße Verfahren parallel oder zeitversetzt zur eigentlichen Produktion von Biogas bzw. der nachfolgenden Strom- oder Wärmeerzeugung durchgeführt wird. Bei dieser Verfahrensvariante ist es weiterhin vorteilhaft, wenn der aus elektrischem Strom erzeugte Wasserstoff zumindest teilweise zur Erzeugung von Biogas, insbesondere im Rahmen einer Methanisierung, eingesetzt wird.

Alternativ ist es selbstverständlich auch möglich, dass eine bisher konventionell genutzte Biogasanlage vollumfänglich zur Durchführung des erfindungsgemäßen Verfahrens umgerüstet wird. Dies ist insbesondere im Fall von kleineren Biogasanlagen, die im landwirtschaftlichen Bereich betrieben werden, von Interesse, weil deren wirtschaftliche Betreibung aufgrund des zumindest in Deutschland unmittelbar bevorstehenden Endes der wirtschaftlichen Förderung solcher Biogasanlagen sehr schwierig bis unmöglich ist.

Ein weiterer Vorteil gegenüber dem konventionellen Betrieb von Biogasanlagen ist im erfindungsgemäßen Verfahren darin zu sehen, dass es sehr sicher betrieben werden kann und eine saisonale Speicherung von Energie möglich ist. Bei konventionellen Biogasanlagen wird das vordergründig erzeugte Biogas in der Regel gleich zur Energieerzeugung in Form von elektrischem Strom und/oder Wärme weiterverarbeitet. Eine Speicherung von Biogas ist schwierig und kostenintensiv, da das in der Regel brennbare Biogas unter erhöhten Sicherheitsvorkehrungen gelagert werden muss. Wird beispielsweise das Biogas unter (sehr) hohem Druck gespeichert, kann dadurch zwar das Speichervolumen des Biogases deutlich reduziert werden, allerdings ist dies in der Regel mit einem hohen Energieverlust und/oder einem großen technischen Aufwand verbunden. Wird umgekehrt herum das Biogas bei niedrigem Druck gespeichert, ist der technische Aufwand hierfür zwar geringer, allerdings sind deutlich größere Speichervolumina der entsprechenden Vorrichtungen erforderlich. Außerdem sind die entsprechenden Biogasreaktoren sowie gegebenenfalls Vorratsbehälter ständig der Gefahr von Gasleckagen ausgesetzt.

Im Gegensatz dazu wird im erfindungsgemäßen Verfahren zunächst Wasserstoff erzeugt, der wiederum selbst ein brennbares Gas darstellt. Allerdings wird der Wasserstoff durch Inkontaktbringen mit einem geeigneten Träger auf sichere Art und Weise gespeichert. Vorzugsweise reagiert der Wasserstoff mit dem Träger in einer chemischen Reaktion, insbesondere in einer Hydrierung, chemisch ab, sodass der Wasserstoff in modifizierter Form sicher gespeichert bzw. gelagert werden kann. Dies bedeutet, dass insbesondere aufgrund der chemischen Reaktion des Wasserstoffs eine Lagerung unter Normaldruck im erfindungsgemäßen Verfahren möglich ist. Die Speicherung des Wasserstoffs an oder im Träger ist jedoch reversibel, sodass im Bedarfsfall, in der Regel nach einer längeren Lagerung, der Wasserstoff wieder problemlos aus dem entsprechenden Träger freigesetzt werden kann. Der freigesetzte Wasserstoff kann anschließend unmittelbar zur Strom- oder gegebenenfalls zur Wärmeerzeugung eingesetzt werden.

Demzufolge kann mit dem erfindungsgemäßen Verfahren Energie über einen längeren Zeitraum sicher zwischengespeichert werden. Dieser Vorteil kann sehr anschaulich anhand der nachfolgenden Fallkonstellation verdeutlicht werden. Beispielsweise wird in günstigen Zeiten ein Überschuss an Energie in Form von elektrischem Strom erzeugt. Denkbar sind hier saisonal verteilte windreiche Tage im Zusammenhang mit der Erzeugung von elektrischem Strom durch Windenergie bzw. sonnenreiche Tage, insbesondere während des Sommerhalbjahres, zur Erzeugung von elektrischem Strom, beispielsweise über Sonnenkollektoren. Der jeweilige Überschuss an Energie, insbesondere an elektrischem Strom, kann erfindungsgemäß in Wasserstoff umgesetzt werden, der wiederum durch Inkontaktbringen mit einem Träger sicher gespeichert sowie über einen längeren Zeitraum gelagert werden kann. Im Bedarfsfall, also bei einem plötzlich eintretenden erhöhten bzw. kontinuierlichen Bedarf an elektrischer Energie, kann der so zwischengespeicherte Wasserstoff bzw. die zwischengespeicherte Energie wieder in elektrischen Strom oder gegebenenfalls in Wärme rückgewandelt bzw. umgewandelt werden.

Ein weiterer Vorteil des erfindungsgemäßen Verfahrens ist darin zu sehen, dass ein Wärmeverbundsystem zusätzlich durchgeführt werden kann. In der Regel ist die Speicherung des Wasserstoffs in oder an dem Träger (T1) eine exotherme Reaktion, bei der somit Energie freigesetzt wird. Dies ist insbesondere der Fall, wenn die Speicherung des Wasserstoffs im Rahmen einer Hydrierung durchgeführt wird. Im Gegensatz dazu ist die Freisetzung des Wasserstoffs aus dem Träger (T2) in der Regel ein endothermer Vorgang, insbesondere wenn dies als Dehydrierung durchgeführt wird. Demzufolge kann die gegebenenfalls zwischengespeicherte Wärme, die bei der exothermen Reaktion anfällt, für die endotherme Rückreaktion bei der Freisetzung des Wasserstoffs wiederverwendet werden. Die Zwischenspeicherung von Wärme kann vorzugsweise in Erdwärmespeichern durchgeführt werden. Die Variante der zwischengespeicherten Wärme ist insbesondere dann vorteilhaft, wenn zwischen der Speicherung des Wasserstoffs durch Inkontaktbringen mit einem Träger (T1) und der nachfolgenden (reversiblen) Freisetzung von Wasserstoff kein allzu langer Zeitraum liegt. So kommen in der Praxis durchaus Fälle vor, wo an günstigen Tagen ein großer Energieüberschuss in Strom bereitsteht, kurz darauf, beispielsweise nach ein oder zwei Tagen oder Wochen, jedoch plötzlich ein Stromengpass vorhanden ist, sodass ein plötzlicher Strombedarf eintritt. Je länger die Zeitspanne zwischen Wasserstoffspeicherung und Wasserstofffreisetzung jedoch beträgt, umso unwirtschaftlicher wird die Variante der Wärmezwischenspeicherung, weil infolge der Zwischenspeicherung mit der Zeit auch Energie/Wärme verloren geht. Denkbar ist in diesem Zusammenhang auch ein plötzlich auftretender Betrieb eines Notfallgenerators zur Stromerzeugung.

Ebenso kann die bei der nachfolgenden Stromerzeugung freiwerdende Wärme zur Durchführung der in der Regel endothermen Freisetzung des Wasserstoffs eingesetzt werden. Außerdem kann die bei der in der Regel exotherm verlaufenden Speicherung des Wasserstoffs freiwerdende Wärme auch sofort im Rahmen des landwirtschaftlichen Betriebs, beispielsweise zur Beheizung von Gewächshäusern, verwendet werden. Weiterhin kann in vorteilhafter Weise die freigesetzte Wärme in einer parallel oder zeitversetzt durchgeführten Erzeugung von Biogas eingesetzt werden.

Nachfolgend wird das erfindungsgemäße Verfahren zum Speichern und Lagern von Wasserstoff sowie das Verfahren zur Erzeugung von elektrischem Strom und gegebenenfalls von Wärme näher erläutert.

Ein erster Gegenstand der vorliegenden Erfindung ist ein Verfahren zum Speichern und Lagern von Wasserstoff, wobei das Verfahren die Schritte a) und b) umfasst.

In Verfahrensschritt a) erfolgt ein Inkontaktbringen eines Trägers (T1) mit Wasserstoff zur Speicherung des Wasserstoffs unter Erhalt eines mit Wasserstoff beladenen Trägers (T2).

Als Träger (T1) kann prinzipiell jeder beliebige, dem Fachmann bekannte Träger verwendet werden, der dazu geeignet ist, Wasserstoff in irgendeiner Form zu speichern. Weiterhin ist erfindungsgemäß der Träger (T1) dazu in der Lage, den Wasserstoff reversibel zu speichern. Dies bedeutet, dass aus dem mit Wasserstoff beladenen Träger (T2), der durch Inkontaktbringen des Wasserstoffs mit dem Träger (T1) erhalten wird, der Wasserstoff zu einem späteren Zeitpunkt (also nach der Lagerung gemäß Verfahrensschritt b)) wieder freigesetzt werden kann und dabei der ursprünglich eingesetzte Träger (T1) wiedergewonnen wird.

Das Speichern des Wasserstoffs kann beispielsweise so durchgeführt werden, dass Wasserstoff chemisch oder physisorptiv, vorzugsweise chemisch, an den Träger (T1) gebunden wird. Im Rahmen der vorliegenden Erfindung ist es jedoch erforderlich, dass das Speichern des Wasserstoffs reversibel möglich ist. Insbesondere Kohlendioxid, das chemisch mit Wasserstoff unter Erhalt von Methan reagieren kann, ist kein Träger (T1) im Sinne der vorliegenden Erfindung, weil aus Methan nicht direkt und/oder auf einfachem Wege reversibel Wasserstoff freigesetzt sowie Kohlendioxid wiedergewonnen werden kann. Folglich ist Methan auch kein mit Wasserstoff beladener Träger (T2) im Sinne der vorliegenden Erfindung.

Eine chemische Bindung des Wasserstoffs an den Träger (T1) kann beispielsweise dadurch erfolgen, dass der Träger (T1) ein Metallhydridspeicher ist, der ein aus einer Metalllegierung hergestelltes Material enthält, das Wasserstoff aufnimmt und chemisch bindet. Alternativ kann der Träger (T1) auch eine, vorzugsweise flüssige, organische Verbindung sein. Solche organischen Verbindungen werden auch als Wasserstoffträger oder LOHC (englisch für: liquid organic hydrogen carriers) bzw. organische Hydride bezeichnet. Solche Wasserstoffträger (LOHC) sind dem Fachmann bekannt und beispielsweise in DE-B 10 2013 223 589, EP-A 1 475 349 und DE-A 10 2012 005 023 beschrieben.

Diese Form der Wasserstoffspeicherung unter Verwendung der LOHCs hat den besonderen Vorteil, dass der LOHC-Träger (T1) in der Regel unter den verwendeten Prozessbedingungen in flüssiger Form vorliegt. Die physiochemischen Eigenschaften eines solchen LOHC-Trägers haben hohe Ähnlichkeit zu herkömmlichen flüssigen Kraftstoffen, sodass Pumpen zum Transport oder Behälter zur Lagerung aus dem Bereich der Kunststoff- und Printstofflogistik genutzt werden können. Die Wasserstoffspeicherung in chemisch gebundener Form in einer organischen Flüssigkeit wie LOHC erlaubt eine drucklose Lagerung bei Normalbedingungen über große Zeiträume ohne signifikanten Wasserstoffverlust. Sofern es sich bei dem Träger (T1) um einen LOHC-Träger handelt, liegt der Träger zur Aufnahme von Wasserstoff in seiner vollständig oder zumindest teilweise dehydrierten Form vor. Vorzugsweise handelt es sich dabei um die vollständig dehydrierte Form. Eine zumindest teilweise dehydrierte Form kann dann vorliegen, wenn beispielsweise eine vollständig dehydrierte Form eines solchen Trägers über zwei oder mehrere Stellen verfügt, an denen das entsprechende Molekül hydriert werden kann, und mindestens eine dieser zur Hydrierung geeigneten Stellen bereits in hydrierter Form vorliegt, aber noch mindestens eine Stelle in dehydrierter (nicht hydrierter) Form vorliegt. Beispiele hierfür sind unter anderem aromatische Verbindungen, insbesondere polyzyklische aromatische Verbindungen mit einem konjugierten π-Elektronensystem.

Das Inkontaktbringen des Trägers (T1) mit Wasserstoff kann prinzipiell nach beliebigen, dem Fachmann bekannten Methoden durchgeführt werden, beispielsweise indem Wasserstoff über den Träger (T1) übergeleitet wird, durch den Träger (T1) durchgeleitet wird oder auf den Träger (T1) aufgepresst wird.

Durch das Inkontaktbringen des Trägers (T1) mit Wasserstoff wird in Schritt a) des erfindungsgemäßen Verfahrens somit ein mit Wasserstoff beladener Träger (T2) erhalten. Sowohl der Träger (T1) als auch der durch Inkontaktbringen mit Wasserstoff erhaltene mit Wasserstoff beladene Träger (T2) können als Gemisch von zwei oder mehreren Trägern (T1) und/oder (T2) vorliegen. Sofern der Wasserstoff chemisch an den Träger (T1) gebunden wird, handelt es sich dabei um eine vollständige oder zumindest teilweise Hydrierung des entsprechenden Trägers (T1), sodass sich der mit Wasserstoff beladene Träger (T2) chemisch vom entsprechenden unbeladenen Träger (T1) unterscheidet. Vorzugsweise handelt es sich dabei um eine vollständige Hydrierung des Trägers (T1) mit Wasserstoff unter Erhalt des mit Wasserstoff beladenen Trägers (T2).

Das Verhältnis der Träger (T1) und (T2) im erfindungsgemäßen Verfahren gemäß Schritt a) wird nachfolgend an dem aus dem Stand der Technik bekannten LOHC-Träger Toluol erläutert. Toluol enthält einen Benzolring und somit drei π-Elektronenpaare. Toluol stellt also einen Träger (T1) dar, der in seiner vollständig dehydrierten Form vorliegt. Durch Inkontaktbringen des Toluols mit Wasserstoff wird erfindungsgemäß eine zumindest teilweise oder vorzugsweise vollständige Hydrierung von Toluol zu Methylcyclohexan als Träger (T2) erzielt. Sofern die vollständige Hydrierung von Toluol durchgeführt wird, werden also alle drei π-Elektronenpaare des Toluols hydriert, sodass Methylcyclohexan die vollständig hydrierte Form des Toluols darstellt. Sofern nur ein oder zwei der π-Elektronenpaare des Toluols hydriert worden sind, handelt es sich nur um eine teilweise Hydrierung. Solche zumindest teilweise hydrierten Verbindungen können sowohl als Träger (T1) oder als Träger (T2) erfindungsgemäß eingesetzt werden.

Normalerweise ist der Träger (T1) eine ungesättigte Verbindung, bevorzugt eine Verbindung mit mindestens einer Kohlenstoff-Kohlenstoff-Doppelbindung, mehr bevorzugt eine aromatische oder hetero-aromatische Verbindung, noch mehr bevorzugt ein N-Alkylcarbazol, Toluol, Dibenzyltoluol, Benzyltoluol, Naphthalin oder ein Azaborin, besonders bevorzugt Dibenzyltoluol.

Bei Verbindungen mit mindestens einer Kohlenstoff-Kohlenstoff-Doppelbindung (C-C-Doppelbindung) kann es sich beispielsweise um ein Olefin, einen Aromaten oder einen Heteroaromaten handeln. Solche Verbindungen sind dem Fachmann prinzipiell bekannt. Diese Verbindungen können gegebenenfalls substituiert sein, beispielsweise durch eine oder mehrere Alkylgruppen von beliebiger Kettenlänge, wie Methyl, Ethyl, Propyl oder durch sonstige funktionelle Gruppen, wie Halogen, Amino oder Carboxyl. Heteroaromatische Verbindungen weisen als Heteroatom mindestens ein Heteroatom auf, das vorzugsweise Sauerstoff, Stickstoff und/oder Schwefel ist. Aromatische oder heteroaromatische Verbindungen können monozyklisch, bizyklisch oder gegebenenfalls polyzyklisch sein. Gegebenenfalls können die entsprechenden Zyklen (Ringe) zumindest teilweise auch in hydrierter Form vorliegen. Ebenso ist es denkbar, dass beispielsweise bei einer bizyklischen Verbindung einer der beiden Zyklen vollständig hydriert ist.

Sofern als Träger (T1) ein N-Alkylcarbazol eingesetzt wird, kann der an das Stickstoffatom gebundene Alkylrest eine beliebige Kettenlänge annehmen, beispielsweise kann es sich dabei um Methyl, Ethyl, Propyl, Isopropyl oder Butyl sowie gegebenenfalls Mischungen davon handeln. Vorzugsweise handelt es sich dabei um N-Ethylcarbazol.

Demzufolge wird im erfindungsgemäßen Verfahren in Schritt a) vorzugsweise die entsprechende teilweise oder insbesondere vollständig hydrierte Form der vorstehend definierten konkreten Träger (T1) als mit Wasserstoff beladener Träger (T2) erhalten.

Prinzipiell ist es möglich, dass Schritt a) in der gleichen Vorrichtung (V1) durchgeführt wird wie der nachfolgend näher definierte Verfahrensschritt b). Dies kann erfolgen, wenn beispielsweise in eine solche Vorrichtung (V1) eine spezielle Einheit/Vorrichtungsbestandteil integriert wird, der die Durchführung des Verfahrensschritts a) getrennt von der Durchführung des Verfahrensschritts b) ermöglicht. Entsprechende Einheiten oder Einbaumaßnahmen sind dem Fachmann bekannt. Vorzugsweise wird jedoch im erfindungsgemäßen Verfahren Schritt a) in einer separaten Vorrichtung (V3) durchgeführt, die vorzugsweise ein chemischer Reaktor ist.

Vorzugsweise wird Schritt a) in einer druckstabilen Vorrichtung, insbesondere in einem druckstabilen chemischen Reaktor, durchgeführt. Weiterhin ist es bevorzugt, dass Schritt a) bei einer Temperatur zwischen 50°C und 400°C, insbesondere zwischen 120°C und 300°C, insbesondere zwischen 150°C und 280°C durchgeführt wird. Die Hydrierung, also das Beladen des Trägers (T1) mit Wasserstoff, findet vorzugsweise bei einem Verfahrensdruck von 2 bar bis 200 bar, insbesondere bei 10 bar bis 100 bar, statt. Weiterhin ist es bevorzugt, dass Schritt a) in Gegenwart eines metallhaltigen Katalysators durchgeführt wird. Als Katalysatoren für die Beladung des LOHC-Trägers (T1) eignen sich insbesondere solche, die das Element Ruthenium und/oder Nickel aufweisen. Es sind auch Katalysatoren möglich, die andere Elemente oder zusätzliche Elemente neben Ruthenium und/oder Nickel aufweisen. Wesentlich sind solche Elemente, die Wasserstoff anlagern und auf LOHC-Träger (T1) übertragen können. Neben Ruthenium und/oder Nickel sind insbesondere Metalle wie Chrom, Eisen, Kobalt, Kupfer, Iridium, Palladium oder Platin als Katalysatoren möglich.

In Verfahrensschritt b) des erfindungsgemäßen Verfahrens erfolgt die Lagerung des mit Wasserstoff beladenen Trägers (T2) in einer Vorrichtung (V1), die Bestandteil einer Biogasanlage ist oder war.

Die Lagerung des mit Wasserstoff beladenen Trägers (T2) kann prinzipiell für beliebige Zeiträume stattfinden. In der Regel erfolgt die Lagerung für mindestens mehrere Tage, sie kann aber auch mehrere Wochen, Monate oder gar Jahre betragen. Genauso ist es aber auch denkbar, dass die Lagerung nur für wenige Minuten oder Stunden oder für einen Tag durchgeführt wird. Vorzugsweise erfolgt die Lagerung für mindestens zwei Tage und/oder maximal sechs Monate.

Als Vorrichtung (V1) kommen prinzipiell alle dem Fachmann bekannten Vorrichtungen in Frage, die Bestandteil einer Biogasanlage sind oder waren. Weiterhin müssen sich die entsprechenden Vorrichtungen (V1) zur Lagerung eines solchen mit Wasserstoff beladenen Trägers (T2) eignen und/oder sie müssen durch, vorzugsweise einfache, Schritte so umgerüstet werden, dass eine entsprechende Lagerung durchgeführt werden kann. Prinzipiell ist es denkbar, dass eine hierfür geeignete Vorrichtung (V1) kommerziell erworben wird und in eine bestehende Biogasanlage eingebaut wird. In anderen Worten ausgedrückt, kann bei dieser Ausführungsform eine neue Vorrichtung (V1) verwendet werden, die vorher noch nicht in einer Biogasanlage eingesetzt gewesen ist und/oder sich für die Verwendung zur Erzeugung von Biogas nicht eignet.

Dies kann erfindungsgemäß insbesondere dann durchgeführt werden, wenn gemäß einer weiter unten beschriebenen bevorzugten Ausführungsform die entsprechende Biogasanlage weiterhin, beispielsweise zeitgleich (parallel) oder zeitversetzt, zur Herstellung von Biogas eingesetzt werden soll. Sofern jedoch eine entsprechende Biogasanlage nicht mehr zur Erzeugung von Biogas eingesetzt werden soll, wird erfindungsgemäß als Vorrichtung (V1) eine Vorrichtung verwendet, die bereits in der entsprechenden Biogasanlage verwendet worden ist. Die letzte Option ist selbstverständlich auch dann denkbar, wenn die entsprechende Anlage weiterhin zur Erzeugung von Biogas verwendet werden soll.

Biogasanlagen als solche sind, wie eingangs bereits erwähnt, dem Fachmann bekannt. In der Regel weisen Biogasanlagen (unter anderem) ein oder mehrere Fermenter, Vorrats- oder Lagerbehälter, Vorrichtungen zur Erzeugung von Strom, insbesondere Gasmotoren, sowie sonstige Leitungen auf. Wie vorstehend bereits erwähnt, kommen als Vorrichtung (V1) prinzipiell alle dem Fachmann bekannten Vorrichtungen in Frage, die Bestandteil einer Biogasanlage sind oder waren. Vorzugsweise handelt es sich dabei um einen Fermenter oder Nachgärer oder um Vorrichtungen, die durch Umrüstung aus einem Fermenter oder Nachgärer hergestellt worden sind. Erfindungsgemäß ist es mehr bevorzugt, dass die Vorrichtung (V1) ein Fermenter ist oder durch Umrüstung aus einem Fermenter hergestellt worden ist. Weiterhin ist es erfindungsgemäß bevorzugt, dass die Vorrichtung (V1) eine separate Vorrichtung ist gegenüber der Vorrichtung, in der der erfindungsgemäße Verfahrensschritt a) durchgeführt wird.

Prinzipiell ist es denkbar, dass die Vorrichtung (V1) weiterhin als Fermenter genutzt wird. Bei dieser Fallkonstellation werden in die Vorrichtung (V1) entsprechende zusätzliche Vorrichtungsbestandteile oder Elemente eingebaut, die eine Lagerung des mit Wasserstoff beladenen Trägers (T2) in der entsprechenden Vorrichtung (V1), insbesondere in einem Fermenter, ermöglichen, ohne dass dabei der bisherige Verwendungszweck im Zusammenhang mit der Herstellung von Biogas eingestellt werden muss. Unter Umständen kann es dabei erforderlich sein, dass weitere Zuleitungen und/oder Ableitungen in die entsprechende Vorrichtung (V1), insbesondere in einen Fermenter, eingebaut werden müssen, um das Einbringen des Trägers (T2) sowie die im Anschluss an die Lagerung erforderliche Entnahme des Trägers (T2) aus der entsprechenden Vorrichtung, insbesondere dem Fermenter, zu ermöglichen.

Erfindungsgemäß ist es jedoch bevorzugt, dass die Vorrichtung (V1), insbesondere ein Fermenter, so umgebaut/umgerüstet wird, dass der bisherige Verwendungszweck im Rahmen der Biogasanlage nicht mehr durchgeführt werden kann. Im Falle eines Fermenters können beispielsweise aus der Vorrichtung (V1) die in einem Fermenter in der Regel vorhandenen Rührwerkzeuge und die damit verbundenen Bedienelemente oder Leitungen ausgebaut werden. Gegebenenfalls vorhandene Heizelemente können hingegen in der entsprechenden Vorrichtung (V1) ohne Probleme belassen werden, da sie auch im Zusammenhang mit der Durchführung des erfindungsgemäßen Verfahrensschritts b) sinnvoll verwendet werden können. Gegebenenfalls können die entsprechenden bisher vorhandenen Zuführ- und/oder Entnahmeelemente in der entsprechenden Vorrichtung (V1) zu den für das erfindungsgemäße Verfahren erforderlichen Zwecken umgerüstet werden oder gegebenenfalls ohne Umrüstung eingesetzt werden.

Vorzugsweise weist die Vorrichtung (V1) zwei räumlich getrennte Bereiche auf, wobei ein erster Bereich zur Lagerung des mit Wasserstoff beladenen Trägers (T2) verwendet wird und ein zweiter Bereich zur Lagerung des Trägers (T1) verwendet wird.

Weiterhin ist es bevorzugt, dass die Vorrichtung (V1) in ihrem Inneren mit einer Schutzauskleidung versehen wird, bevor Schritt b) und gegebenenfalls die Lagerung des Trägers (T1) durchgeführt wird.

Weiterhin ist es bevorzugt, dass in die Vorrichtung (V1) eine schwimmende Trennwand eingebaut ist und der mit Wasserstoff beladene Träger (T2) unterhalb der schwimmenden Trennwand gelagert wird.

Weiterhin ist es bevorzugt, dass die Vorrichtung (V1) Bestandteil einer Biogasanlage war und von allen Substanzen gereinigt war, die sich infolge der Erzeugung von Biogas in ihrem Inneren oder gegebenenfalls in ihren Zuleitungen befunden haben, bevor Schritt b) und gegebenenfalls die Lagerung des Trägers (T1) durchgeführt wird.

In einer bevorzugten Ausführungsform der vorliegenden Erfindung wird die Vorrichtung (V1), die Bestandteil einer Biogasanlage ist oder war, vor Durchführung des Verfahrensschritts b) einem Reinigungsschritt f) unterzogen. Bei dieser Ausführungsform handelt es sich bei der Vorrichtung (V1) vorzugsweise um einen Fermenter (F1) oder einen Nachgärer. Im nachfolgenden Text wird diese Ausführungsform anhand des Fermenters (F1) erklärt. Sinngemäße Aussagen gelten aber genauso für alle sonstigen Vorrichtungen (V1), die kein Fermenter sind.

Das Reinigen kann prinzipiell nach allen dem Fachmann bekannten Methoden durchgeführt werden. Die konkrete Durchführung des Reinigungsschritts f) hängt unter anderem auch von der Art und Größe des zu reinigenden Fermenters (F1) ab und/oder von der Dauer sowie den sich im Fermenter befindlichen Substanzen, die zur Erzeugung von Biogas verwendet worden sind. Beispielsweise kann es sich, wie eingangs erwähnt, bei den zur Erzeugung von Biogas verwendeten Substanzen auch um Gülle oder Festmist handeln, die beispielsweise ätzend sein können und somit bei einer langen Betriebsdauer das Innere eines Fermenters beschädigen und/oder kontaminieren können. Ist beispielsweise ein Fermenter nicht lange zur Erzeugung von Biogas in Betrieb gewesen und/oder wurden relativ ungefährliche bzw. nicht oder geringfügig ätzende Stoffe zur Erzeugung von Biogas in dem Fermenter verwendet, kann es erfindungsgemäß ausreichend sein, wenn das Reinigen des Inneren des Fermenters (F1) durch technisch bzw. zeitmäßig relativ einfache Reinigungsschritte durchgeführt wird, wie beispielsweise die nachfolgend definierten Teilschritte f2) oder f4). Diese Schritte können zur Reinigung beispielsweise auch dann ausreichend sein, sofern ein Fermenter (F1) in seinem Inneren vollständig aus Edelstahl hergestellt ist.

War hingegen ein Fermenter (sehr) lange zur Erzeugung von Biogas im Einsatz und/oder wurden als Substanzen relativ ätzende Komponenten, wie Gülle oder Festmist, eingesetzt, ist es hingegen vorteilhaft, wenn der Verfahrensschritt f) die Durchführung des nachfolgend definierten Teilschritts f1) umfasst. Dies ist insbesondere der Fall, wenn Teile des Inneren des Fermenters, insbesondere die Wände und/oder der Boden nicht aus Edelstahl, sondern beispielsweise aus Beton oder herkömmlichem Stahl hergestellt sind. Sofern im Inneren des Fermenters (F1) weitere Vorrichtungselemente, wie Heizungen oder sonstige Leitungen, die zur Erzeugung des Biogases verwendet worden sind, belassen werden, können diese Vorrichtungselemente sinngemäßen Reinigungsschritten unterzogen werden.

Vorzugsweise umfasst der Schritt f) einen Teilschritt f1), wobei vom Inneren des Fermenters (F1) mindestens eine Oberflächenschicht zumindest teilweise entfernt wird. Das Entfernen der Oberflächenschicht kann hierbei nach allen dem Fachmann bekannten Methoden erfolgen. Vorzugsweise erfolgt das Entfernen durch Abschleifen oder Abtragen, insbesondere durch Abschleifen, von mindestens einer Oberflächenschicht. Besonders bevorzugt sind dabei eine abrasive Hochdruckreinigung und/oder die Verwendung von Sandstrahlern. Die Oberfläche im Inneren des Fermenters (F1) kann dabei prinzipiell in beliebigen Dicken und/oder Anzahl von Schichten entfernt, vorzugsweise abgeschliffen, werden. Vorzugsweise erfolgt das Entfernen jedoch nur bis zu einer Schichtdicke von ein paar Millimetern, beispielsweise 1 bis 10 mm, vorzugsweise 2 bis 5 mm. Das Entfernen der Oberflächenschicht erfolgt vorzugsweise vollständig, insbesondere von den Wänden und/oder dem Boden im Inneren des Fermenters (F1). Weiterhin ist es bevorzugt, dass Teilschritt f) an den Bereichen im Inneren des Fermenters (F1) durchgeführt wird, die aus Beton, Stahl oder gegebenenfalls aus Stahlbeton hergestellt worden sind. Sofern das Innere des Fermenters aus Stahlbeton hergestellt ist, wird Teilschritt f) nur soweit durchgeführt, dass die Armierung des Stahlbetons und/oder die die Armierung enthaltende Schicht des Stahlbetons nicht entfernt wird.

Weiterhin kann Schritt f) einen Teilschritt f2) umfassen, wobei im Inneren des Fermenters (F1) mindestens eine alkalische Wäsche durchgeführt wird. Die Durchführung der alkalischen Wäsche erfolgt prinzipiell nach dem Fachmann bekannten Methoden. Der Teilschritt f2) kann gegebenenfalls auch mehrfach wiederholt werden und/oder durch eine Wäsche im neutralen Bereich, also unter Verwendung von Wasser ohne alkalische Zusätze, unterbrochen werden. Zur alkalischen Wäsche können alle dem Fachmann bekannten Chemikalien verwendet werden, die einen pH-Wert im Bereich > 7, bevorzugt 8 bis 14, insbesondere 9 bis 13, ermöglichen. Beispielsweise können hierfür auch Natronlauge oder sonstige Basen verwendet werden, deren genauer pH-Wert durch die Zugabe von Wasser oder gegebenenfalls von Puffern eingestellt wird.

Weiterhin kann Schritt f) einen Teilschritt f3) umfassen, wobei aus dem Inneren des Fermenters (F1) sowie gegebenenfalls aus seinen Zuleitungen alle Substanzen, die sich infolge der Erzeugung von Biogas im Inneren des Fermenters (F1) oder gegebenenfalls in seinen Zuleitungen befinden, entfernt werden. Vorzugsweise erfolgt das Entfernen gemäß Teilschritt f3) durch Ablassen, vorzugsweise durch Absaugen, der Substanzen. Der Teilschritt f3) wird erfindungsgemäß vorzugsweise immer dann durchgeführt, sofern noch Substanzen von der zuvor durchgeführten Erzeugung von Biogas im Inneren des Fermenters bzw. seinen Zuleitungen vorhanden sind.

Weiterhin kann Schritt f) auch einen Teilschritt f4) umfassen, in dem das Innere des Fermenters (F1) mit Wasser oder einem wässrigen Reinigungsmittel gespült wird. Im Anschluss daran kann gegebenenfalls noch ein Trocknungsschritt durchgeführt werden. Vorzugsweise umfasst Schritt f) einen Teilschritt f4), wobei im Anschluss an mindestens einen der Teilschritte f1) bis f3) mit Wasser oder einem wässrigen Reinigungsmittel gespült sowie gegebenenfalls danach ein Trocknungsschritt durchgeführt wird.

Weiterhin ist es bevorzugt, dass
i) von Schritt f) die Teilschritte f1) und f2) durchgeführt werden, oder
ii) von Schritt f) die Teilschritte f1), gefolgt von f2) und gefolgt von f4) durchgeführt werden, wobei gegebenenfalls vor Durchführung des Teilschritts f1) der Teilschritt f3) durchgeführt wird, oder
iii) der Teilschritt f3) zusätzlich vor einem der Teilschritte f1), f2) oder f4), vorzugsweise vor Teilschritt f1), durchgeführt wird.

Weiterhin ist es bevorzugt, dass zur Reinigung der Decke des Fermenters (F1) der Teilschritt f2) und/oder der Teilschritt f4) durchgeführt wird. Ebenso ist es bevorzugt, dass die Reinigung einer Zuleitung des Fermenters (F1) bis zur ersten Absperrvorrichtung der Zuleitung erfolgt und/oder eine Zuleitung des Fermenters (F1) im Inneren des Fermenters (F1) verschlossen wird, vorzugsweise durch Anbringen eines Stopfens. Ebenso ist es bevorzugt, dass ein im Inneren des Fermenters (F1) vorhandenes Rührwerkzeug vor Durchführung von einem der Teilschritte f1), f2) oder f4) und/oder nach Durchführung des Teilschritts f3) ausgebaut wird.

In einer weiteren bevorzugten Ausführungsform kann im Anschluss an den vorstehend beschriebenen Verfahrensschritt f) ein Verfahrensschritt g) durchgeführt werden, wobei im Inneren der Vorrichtung (V1) mindestens eine Schutzauskleidung angebracht wird. Die Vorrichtung (V1) ist in dieser Ausführungsform vorzugsweise ein Fermenter (F1) oder ein Nachgärer. Die Ausführungsform wird nachfolgend anhand des Fermenters (F1) erklärt. Diese Aussagen gelten jedoch genauso für alle anderen Vorrichtungen (V1), die kein Fermenter oder Nachgärer sind. Sofern der optionale Verfahrensschritt g) durchgeführt wird, erfolgt dies vor Durchführung von Verfahrensschritt b).

Das Anbringen der Schutzauskleidung kann prinzipiell nach allen dem Fachmann bekannten Methoden erfolgen, beispielsweise durch Aufkleben oder eine mechanische Befestigung wie Festnageln, Anschrauben oder Verspannen. Hierfür geeignete Hilfsmittel, wie Klebstoffe, Schrauben oder Nägel sind dem Fachmann bekannt. Sofern Klebstoffe verwendet werden, können hierfür prinzipiell alle bekannten Klebstoffe verwendet werden, wie anorganische oder organische Klebstoffe, beispielsweise auf Metalloxiden basierende Kleber oder Polyurethanklebstoffe.

Im Rahmen der vorliegenden Erfindung ist es bevorzugt, dass Schritt g) durchgeführt wird. Schritt g) wird insbesondere dann durchgeführt, wenn das Innere des Fermenters (F1) vollständig oder zumindest teilweise, vorzugsweise vollständig, nicht aus Edelstahl hergestellt ist. Besonders bevorzugt wird Schritt g) dann durchgeführt, sofern die Wände und/oder der Boden im Inneren des Fermenters (F1) aus Beton hergestellt sind. Vorzugsweise wird Schritt g) hingegen nicht durchgeführt, sofern die Wände und/oder der Boden im Inneren des Fermenters (F1) aus Edelstahl hergestellt sind. Weiterhin ist es bevorzugt, dass ein im Inneren des Fermenters (F1) vorhandenes Rührwerkzeug vor Durchführung von Schritt b) oder gegebenenfalls vor Durchführung von Schritt g) ausgebaut wird.

Als Schutzauskleidung können prinzipiell alle dem Fachmann bekannten Schutzauskleidungen verwendet werden, beispielsweise in Form von Folien, als sogenannte Liner oder als Beschichtungen aus Epoxidharzen. Die Schutzauskleidung kann im Inneren des (gereinigten) Fermenters (F1) so angebracht werden, dass sie vollständig oder zumindest teilweise das komplette Innere des Fermenters (F1) bedeckt. Vorzugsweise werden diejenigen Bereiche des (gereinigten) Fermenters (F1) mit Schutzauskleidung bedeckt, die aus Beton oder Stahl sowie Stahlbeton hergestellt worden sind. Ist beispielsweise das Dach im Inneren des Fermenters (F1) aus Kunststoff hergestellt, ist es nicht erforderlich, solche aus Kunststoff hergestellten Bereiche mit einer zusätzlichen Schutzauskleidung zu versehen. Gegebenenfalls kann dies aber trotzdem gemacht werden. Vorzugsweise bedeckt die Schutzauskleidung den Boden und/oder die Wände im Inneren des gereinigten Fermenters (F1) vollständig oder zumindest größtenteils.

Weiterhin ist es bevorzugt, dass die Schutzauskleidung aus mindestens einem Kunststoff hergestellt ist, vorzugsweise ist der Kunststoff ausgewählt aus einem Polyalkylen, einem Epoxidharz, einem Polyurethan und/oder einer Plastikfolie, mehr bevorzugt ist der Kunststoff ausgewählt aus einem Polyethylen (PE), einem HD (high density)-Polyethylen (HD-PE), einem Polypropylen (PP) und/oder einem Polyurethan (PU).

Weiterhin ist es bevorzugt, dass die Schutzauskleidung mit Glasfasern verstärkt ist, vorzugsweise ist die Schutzauskleidung aus mindestens einem glasfaserverstärkten Kunststoff hergestellt. Die Schutzauskleidung kann gegebenenfalls mit Glasfasern verstärkt sein. Sofern dies der Fall ist, ist die Schutzauskleidung bevorzugt aus mindestens einem glasfaserverstärkten Kunststoff hergestellt.

Die Schutzauskleidung selbst kann wiederum genau eine Schicht aufweisen, gegebenenfalls kann sie auch zwei oder mehrere Schichten umfassen. Sofern die Schutzauskleidung einschichtig ist, erfolgt das Anbringen der Schutzauskleidung im Inneren des gereinigten Fermenters (F1) vorzugsweise durch Verkleben. Sofern sie zwei- oder mehrschichtig ist, erfolgt das Anbringen vorzugsweise im Rahmen einer mechanischen Befestigung, insbesondere durch Verspannen. Prinzipiell ist es denkbar, dass im Inneren des Fermenters (F1) in unterschiedlichen Bereichen unterschiedliche Arten von Schutzauskleidungen angebracht werden. Gegebenenfalls können auch mehrere Schutzauskleidungen übereinander angebracht werden. Vorzugsweise wird im Inneren des Fermenters (F1) eine Art von Schutzauskleidung einheitlich verwendet, wobei die Schutzauskleidung gegebenenfalls auch aus mehreren Schichten aufgebaut sein kann.

Weiterhin kann im Rahmen dieser Ausführungsform als Variante die Schutzauskleidung aus mindestens zwei Schichten aufgebaut und gegebenenfalls mit einer zusätzlichen Druckkontrolleinrichtung versehen sein, vorzugsweise befindet sich ein Gas, insbesondere Luft, zwischen zwei Schichten der Schutzauskleidung. Weiterhin ist es bevorzugt, dass das Gas unter Druck ist. Der Druck kann beliebige Werte annehmen, vorzugsweise beträgt der Druck des Gases 1,5 bis 10 bara (bar absolut). Der Druck kann über eine permanente Druckmessung überwacht werden. So kann eine potentielle Leckage der inneren oder äußeren Schutzauskleidung frühzeitig in Form eines Druckabfalls erkannt werden, ohne dass das Trägermedium aus dem Behälter lecken kann.

Weiterhin kann im Rahmen dieser Ausführungsform als Variante eine mindestens zwei Schichten aufweisende Schutzauskleidung, die eine Schicht aus Polyethylen und eine Schicht aus HD-Polyethylen umfasst, so an die Wände und/oder den Boden im Inneren des Fermenters (F1) angebracht sein, dass die Schicht aus HD-Polyethylen sich auf der von den Wänden und/oder dem Boden abgewandten Seite der Schutzauskleidung im Inneren des Fermenters (F1) befindet.

Weiterhin können im Rahmen dieser Ausführungsform als Variante die Wände und/oder der Boden im Inneren des Fermenters (F1) aus Beton hergestellt sein, und eine Schutzauskleidung, die aus einem Polypropylen oder einem Polyurethan hergestellt ist, wird so auf den Beton aufgebracht, dass die Schicht der Schutzauskleidung, die aus einem Polypropylen oder einem Polyurethan hergestellt ist, direkt an den Beton angebracht ist. Sofern bei dieser Ausführungsform die Schutzauskleidung durch Kleben angebracht wird, bedeutet dies, dass sich der Klebstoff zwischen Schutzauskleidung und Boden oder Wand befindet.

Weiterhin können im Rahmen dieser Ausführungsform als Variante in das Innere des gereinigten Fermenters (F1) zwei räumlich getrennte Bereiche eingebaut werden, wobei vorzugsweise die räumliche Trennung durch Einbau einer schwimmenden Trennwand in horizontaler Richtung oder durch Einbau einer Trennwand in vertikaler Richtung im Inneren des Fermenters (F1) erfolgt, wobei die Trennwand gegebenenfalls mit mindestens einer Schutzauskleidung versehen ist, besonders bevorzugt erfolgt die räumliche Trennung durch Einbau einer Trennwand in vertikaler Richtung im Inneren des Fermenters (F1), wobei die Trennwand gegebenenfalls mit mindestens einer Schutzauskleidung versehen ist.

Der nachfolgend beschriebene optionale Verfahrensschritt c) sowie der vorstehend beschriebene Schritt a) können zeitgleich, vor oder auch nach den vorstehend beschriebenen optionalen Verfahrensschritten f) und g) durchgeführt werden. Die Durchführung der Verfahrensschritte f) und g) einerseits sowie c) und a) andererseits ist prinzipiell vollkommen unabhängig voneinander. Insbesondere bei den Verfahrensschritten c) und a) handelt es sich um Verfahrensschritte, die auch mehrmals und/oder kontinuierlich durchgeführt werden können, während hingegen die Verfahrensschritte f) und g) vorzugsweise nur einmal im Rahmen des konkreten Umrüstungsvorganges einer Biogasanlage bzw. eines Fermenters durchgeführt werden. Die Anzahl sowie konkrete Ausgestaltung der Durchführung der Verfahrensschritte c) und a) hängt hingegen mehr von der Art und Anzahl der Durchführung der dem Verfahrensschritt b) nachgelagerten Verfahrensschritte d) sowie e) ab, also der Erzeugung von Strom unter Verwendung des in Verfahrensschritt b) gelagerten Wasserstoffs.

Im erfindungsgemäßen Verfahren ist es bevorzugt, dass vor Schritt a) ein Schritt c) durchgeführt wird. Gemäß des optionalen erfindungsgemäßen Schritts c) erfolgt die Erzeugung von Wasserstoff in einer Vorrichtung (V6), vorzugsweise in einem Elektrolyseur, unter Verwendung von Wasser und elektrischem Strom. Die Durchführung des Verfahrensschritts c) als solchem ist dem Fachmann bekannt. Geeignete Elektolyseure sind beispielsweise in DE-B 10 2013 223 589 oder DE-A 10 2012 005 023 beschrieben. Hierbei kann es sich beispielsweise um einen sogenannten PEM-Elektrolyseur (Polymerelektrolytmembran) oder um einen SOEC-Elektrolyseur (solid oxide electolysis cell) sowie um einen Hochtemperatur-Elektrolyseur handeln. Letztendlich ist als Vorrichtung (V6) jede Art von Vorrichtung geeignet, in der die Zerlegung von Wasser in Wasserstoff oder Sauerstoff bei gleichzeitiger Einspeisung von elektrischem Strom durchgeführt werden kann. In der Vorrichtung (V6) sind in der Regel zwei Elektroden vorhanden. Bei dem eingesetzten Wasser handelt es sich normalerweise um leitfähiges Wasser, das gegebenenfalls weitere Zusätze enthalten kann, um die Wasserelektrolyse zu ermöglichen.

Der in Schritt c) verwendete elektrische Strom kann prinzipiell aus beliebigen Quellen stammen. So ist es denkbar, dass der elektrische Strom einem separaten Netz (Verbrauchernetz) entnommen wird. Vorzugsweise handelt es sich jedoch bei dem eingesetzten Strom gemäß Schritt c) um regenerativ hergestellten elektrischen Strom (Elektrizität). Dieser regenerativ hergestellte elektrische Strom wird vorzugsweise direkt oder in unmittelbarer Nähe der Durchführung des erfindungsgemäßen Verfahrens erzeugt. Der elektrische Strom kann beispielsweise aus Wasserkraft, Windenergie, solarer Strahlung (Sonnenenergie), Erdwärme (Geothermie), Biogas, Bioethanol, Holz oder durch die Gezeiten gewonnen werden. Die zur Gewinnung des elektrischen Stroms notwendigen Maschinen und Apparate sind ebenfalls bekannt. Es kann sich dabei um Wasserturbinen, Windanlagen, Fotovoltaik, Solarthermie, Verbrennungsmaschinen oder Turbinen, bei deren Brennstoff es sich um Biomasse handelt, stammen. Vorzugsweise wird der in Schritt c) des erfindungsgemäßen Verfahrens eingesetzte elektrische Strom durch Wasserkraft, Windenergie oder solare Strahlung erzeugt. Da dieser elektrische Strom prinzipiell auch aus von nachwachsenden Rohstoffen gewonnener Biomasse, insbesondere Biogas, stammen kann, ist es auch denkbar, dass das in einer Ausführungsform der vorliegenden Erfindung parallel oder zeitversetzt hergestellte Biogas in einem nachfolgenden Schritt verstromt wird und dieser Strom, für den aktuell keine Verwendung besteht, im Rahmen des erfindungsgemäßen Verfahrens in Wasserstoff umgewandelt wird, der erfindungsgemäß gespeichert und gelagert wird.

Wie vorstehend bereits erwähnt, wird in Schritt c) (in der Regel) neben Wasserstoff zusätzlich Sauerstoff erzeugt. Vorzugsweise wird in Schritt c) zusätzlich Sauerstoff erzeugt und dieser Sauerstoff wird in der (nachfolgend definierten) Vorrichtung (V2) gemäß Schritt e) gemeinsam mit Wasserstoff in elektrischen Strom und gegebenenfalls Wärme umgewandelt, wobei der Sauerstoff vor seiner Verwendung in Schritt e) gegebenenfalls zwischengespeichert wird. Vorrichtungen zum Zwischenspeichern von Sauerstoff sind dem Fachmann bekannt.

Weiterhin ist es bevorzugt, dass in Schritt c) zusätzlich Sauerstoff erzeugt wird und dieser Sauerstoff zur Optimierung der Haltung von Masttieren in einem landwirtschaftlichen Betrieb, zur Beschleunigung des Pflanzenwachstums in einem landwirtschaftlichen Betrieb oder zur Ozonisierung bei der Trinkwasserreinigung verwendet wird.

Weiterhin ist es bevorzugt, dass in Schritt c) zusätzlich Wärme frei wird, und die freigewordene Wärme gegebenenfalls in einem landwirtschaftlichen Betrieb verwendet wird oder nach (dem nachfolgend definierten) Schritt d) geleitet wird, vorzugsweise wird die in Schritt c) freigesetzte Wärme zwischengespeichert, insbesondere in einem Erdwärmespeicher, bevor sie nach Schritt d) geleitet wird. Vorrichtungen zur Zwischenspeicherung von Wärme als solche sind dem Fachmann bekannt. Für die Errichtung eines Erdwärmespeichers kann in vorteilhafter Weise ein in einer Biogasanlage vorhandenes Substratlager oder Substrattank verwendet werden. Dies gilt für jeden Teilschritt im Rahmen der vorliegenden Erfindung, bei dem Wärme in irgendeiner Form zwischengespeichert werden soll.

Weiterhin ist es bevorzugt, dass im erfindungsgemäßen Verfahren nach Schritt b) ein Schritt d) durchgeführt, wobei gemäß dem optionalen Verfahrensschritt d) die Freisetzung von Wasserstoff aus dem mit Wasserstoff beladenen Träger (T2) erfolgt.

Prinzipiell ist es möglich, dass der optionale Verfahrensschritt d) ebenfalls in der Vorrichtung (V1) durchgeführt wird. Vorzugsweise wird der Verfahrensschritt d) in einer separaten Vorrichtung (V4) durchgeführt, die insbesondere ein chemischer Reaktor ist. Die erfindungsgemäßen Verfahrensschritte a), b) und d) werden also vorzugsweise jeweils in separaten Vorrichtungen durchgeführt. Prinzipiell ist es möglich, dass die Vorrichtung (V4) zur Durchführung des Schritts d) in einer von der Art her gleichen Vorrichtung durchgeführt wird wie die Vorrichtung (V3) zur Durchführung des erfindungsgemäßen Verfahrensschritts a). Geeignete Vorrichtungen zur Durchführung des erfindungsgemäßen Verfahrensschritts d) sind beispielsweise in DE-B 10 2013 223 589 offenbart.

Das Freisetzen des Wasserstoffs gemäß Verfahrensschritt d) erfolgt vorzugsweise in einem druckstabilen chemischen Reaktor bei einer Prozesstemperatur zwischen 100°C und 450°C, bevorzugt zwischen 150°C und 420°C und insbesondere zwischen 180°C und 390°C. Der Prozessdruck liegt zwischen 0,1 und 30 bar, insbesondere zwischen 1 und 10 bar, wobei insbesondere ein metallhaltiger Katalysator eingesetzt werden kann, der insbesondere Platin und/oder Palladium enthält. Wesentlich ist, dass der Katalysator geeignet ist, Wasserstoff, der vom LOHC-Träger (T2) abgegeben wird, als Wasserstoffgas freizusetzen. Neben Platin und/oder Palladium sind dafür insbesondere Metalle, wie Chrom, Eisen, Kobalt, Nickel, Kupfer, Iridium oder Ruthenium, geeignet.

Weiterhin ist es bevorzugt, dass bei der Freisetzung von Wasserstoff gemäß Schritt d) zusätzlich der Träger (T1) wiedergewonnen wird.

Weiterhin ist es bevorzugt, dass bei der Freisetzung von Wasserstoff gemäß Schritt d) zusätzlich der Träger (T1) wiedergewonnen wird und der Träger (T1) erneut zur Durchführung von Schritt a) verwendet wird.

Weiterhin ist es bevorzugt, dass bei der Freisetzung von Wasserstoff gemäß Schritt d) zusätzlich der Träger (T1) wiedergewonnen wird und anschließend der Träger (T1) in einer separaten Vorrichtung (V5) oder in der Vorrichtung (V1) gelagert wird.

Weiterhin ist es erfindungsgemäß bevorzugt, dass nach Schritt d) ein optionaler Verfahrensschritt e) durchgeführt wird, wobei in Schritt e) die Überführung des in Schritt d) erhaltenen Wasserstoffs in eine Vorrichtung (V2) erfolgt, wobei in der Vorrichtung (V2) Wasserstoff in elektrischen Strom und gegebenenfalls in Wärme umgewandelt wird.

Die Vorrichtung (V2) als solche ist dem Fachmann bekannt. Vorzugsweise ist die Vorrichtung (V2) Bestandteil eines Blockheizkraftwerkes, ein Gasmotor, eine Brennstoffzelle, ein Verbrennungsmotor, ein Verbrennungsmotor mit angeschlossenem Generator, eine Turbine oder eine Wasserstoffturbine mit angeschlossenem Generator, vorzugsweise ein Gasmotor.

Die vorgenannten Vorrichtungen (V2) sind häufig bereits in einer konventionellen Biogasanlage enthalten und können in aller Regel ohne weitere Umrüstungen im erfindungsgemäßen Verfahren eingesetzt werden. Dies trifft insbesondere für ein Blockheizkraftwerk und/oder einen Gasmotor zu, die häufig in Biogasanlagen verwendet werden. Weiterhin ist es bevorzugt, dass die gegebenenfalls in Schritt e) erzeugte Wärme nach Schritt d) zurückgeleitet wird. Die in Schritt e) freigesetzte Wärme kann gegebenenfalls mit der, wie vorstehend bereits erwähnt, in Schritt c) optional anfallenden Wärme vereinigt und gegebenenfalls gemeinsam zwischengelagert werden. Sinngemäßes trifft auch auf die Wärme zu, die zusätzlich im erfindungsgemäßen Verfahren in Schritt a) freiwerden kann.

Vorzugsweise wird in Schritt a) zusätzlich Wärme frei und diese freigewordene Wärme wird in einem landwirtschaftlichen Betrieb verwendet, vorzugsweise zur Beheizung von Gewächshäusern. Ebenso ist es bevorzugt, dass in Schritt a) zusätzlich Wärme frei wird und die freigewordene Wärme nach Schritt d) geleitet wird, wobei die Wärme vor ihrer Verwendung in Schritt d) vorzugsweise zwischengespeichert wird, insbesondere in einem Erdwärmespeicher zwischengespeichert wird.

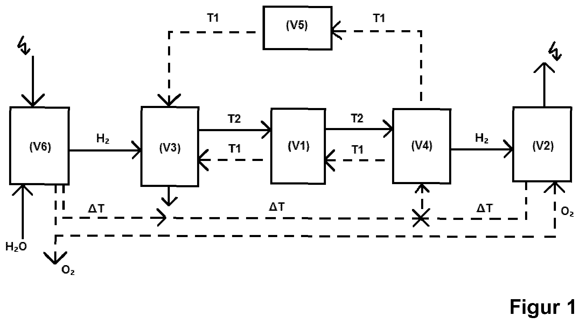

Figur 1 zeigt eine bevorzugte Ausführungsform des erfindungsgemäßen Verfahrens. Die in Figur 1 gestrichelt dargestellten Linien bzw. Pfeile stellen optionale Ausgestaltungen dieser bevorzugten Ausführungsform dar. In dieser Ausführungsform wird zunächst in der räumlich von der Vorrichtung (V1) getrennten Vorrichtung (V3), die vorzugsweise ein chemischer Reaktor ist, Wasserstoff mit einem geeigneten Träger (T1) in Kontakt gebracht, wodurch der Wasserstoff gespeichert wird, vorzugsweise durch chemische Bindung an den Träger (T1), insbesondere durch Hydrierung, wodurch ein mit Wasserstoff beladener Träger (T2) erzeugt wird. Dies entspricht Schritt a) des erfindungsgemäßen Verfahrens.

Der so erzeugte, mit Wasserstoff beladene Träger (T2) wird in die Vorrichtung (V1) überführt und dort gelagert, gemäß Schritt b) des erfindungsgemäßen Verfahrens. Bei der Vorrichtung (V1) handelt es sich vorzugsweise um einen Fermenter, der zuvor in einer Biogasanlage eingesetzt wurde und der gegebenenfalls umgerüstet worden ist, um im erfindungsgemäßen Verfahren eingesetzt zu werden. Die Lagerung des mit Wasserstoff beladenen Trägers (T2) in der Vorrichtung (V1) kann für einen beliebigen Zeitraum erfolgen. Weiterhin ist es bevorzugt, dass in dieser Ausführungsform in der Vorrichtung (V1) auch der Träger (T1) gelagert werden kann, um ihn im Bedarfsfall in Schritt a) des erfindungsgemäßen Verfahrens einsetzen zu können.

Der in Schritt a) eingesetzte Wasserstoff wird in dieser Ausführungsform in einer Vorrichtung (V6) erzeugt, bei der es sich vorzugsweise um einen Elektrolyseur handelt. In der Vorrichtung (V6) wird elektrischer Strom, der vorzugsweise aus erneuerbaren Energien, insbesondere aus Windenergie oder aus Sonnenkollektoren, erzeugt worden ist, in Gegenwart von Wasser in Wasserstoff sowie Sauerstoff umgewandelt. Der in der Vorrichtung (V6) ebenfalls anfallende Sauerstoff kann gegebenenfalls im erfindungsgemäßen Verfahren in die nachfolgend beschriebene Vorrichtung (V2) zur Rückgewinnung von Strom eingesetzt werden, gegebenenfalls kann der Sauerstoff auch zu anderen Zwecken verwendet werden, beispielsweise zur Förderung des Pflanzenwachstums oder der Haltung von Masttieren. Die in der Vorrichtung (V6) und/oder in der Vorrichtung (V3) gegebenenfalls freiwerdende Wärme kann im erfindungsgemäßen Verfahren ebenfalls wiedergewonnen werden, insbesondere im Zusammenhang mit der unten beschriebenen Vorrichtung (V4).

In dieser Ausführungsform der vorliegenden Erfindung wird im Bedarfsfall, also wenn ein erhöhter Bedarf an Stromerzeugung vorhanden ist, der in der Vorrichtung (V1) (zwischen)gelagerte Wasserstoff in Form des mit Wasserstoff beladenen Trägers (T2) gemäß Schritt d) in die Vorrichtung (V4) überführt, wo Wasserstoff wiederum aus dem mit Wasserstoff beladenen Träger (T2) freigesetzt wird. Diese in der Regel endotherm verlaufende Reaktion der Freisetzung von Wasserstoff sowie als Nebenprodukt des Trägers (T1) kann erfindungsgemäß durch Wärmeeintrag, insbesondere von Wärme aus der unten beschriebenen Vorrichtung (V2) sowie gegebenenfalls aus den Vorrichtungen (V3) sowie (V6), gesteuert werden.

Der freigesetzte Wasserstoff wird in dieser Ausführungsform erfindungsgemäß gemäß Schritt e) in die Vorrichtung (V2) überführt, um dort in elektrischen Strom und gegebenenfalls in Wärme umgewandelt zu werden. Vorzugsweise handelt es sich bei der Vorrichtung (V2) um einen Bestandteil eines Blockheizkraftwerkes, insbesondere um einen Gasmotor. Gegebenenfalls kann in der Vorrichtung (V2) zusätzlich Sauerstoff zur Stromerzeugung eingesetzt werden.

Die bei der Stromerzeugung in der Vorrichtung (V2) anfallende Wärme wird vorzugsweise in das Verfahren rückgeleitet, insbesondere in die Vorrichtung (V4), in der die in der Regel endotherm verlaufende Freisetzung des Wasserstoffs aus dem Träger (T2) durchgeführt wird. Der in der Vorrichtung (V4) ebenfalls anfallende "freigesetzte" Träger (T1) kann, wie vorstehend bereits erwähnt, entweder in der Vorrichtung (V1) gelagert werden oder in einer separaten Vorrichtung (V5). Bei der Vorrichtung (V5) kann es sich auch um einen Fermenter einer Biogasanlage handeln. Im Bedarfsfall, also wenn wiederum Wasserstoff zwischengespeichert und gelagert werden soll, kann der in den Vorrichtungen (V1) und/oder (V5) zwischengelagerte Träger (T1) in die Vorrichtung (V3) rückgeführt werden, um wiederum einen mit Wasserstoff beladenen Träger (T2) zu erzeugen.

In einer weiteren bevorzugten Ausführungsform der vorliegenden Erfindung wird das Verfahren in einer Anlage durchgeführt, die weiterhin zur Erzeugung von Biogas eingesetzt werden kann. Die Erzeugung von Biogas kann dabei parallel oder zeitversetzt zum erfindungsgemäßen Verfahren zum Speichern und Lagern von Wasserstoff bzw. bei der gegebenenfalls nachfolgend durchgeführten Freisetzung von Wasserstoff und der damit verbundenen Erzeugung von elektrischem Strom und gegebenenfalls von Wärme durchgeführt werden. Ebenso ist es denkbar, dass die Erzeugung von Biogas nur für ein bestimmtes Zeitintervall parallel zum Verfahren zum Speichern und Lagern von Wasserstoff durchgeführt wird.

Vorzugsweise werden die Erzeugung von Biogas einerseits und das Speichern und Lagern von Wasserstoff oder die Erzeugung von elektrischem Strom sowie gegebenenfalls von Wärme andererseits parallel auf derselben Anlage durchgeführt.

Weiterhin ist es bevorzugt, dass die Erzeugung von Biogas in einem separaten Fermenter durchgeführt wird, der nicht Bestandteil der Vorrichtung (V1) zur Lagerung des mit Wasserstoff beladenen Trägers (T2) ist.

Weiterhin ist es bevorzugt, dass der in Schritt c) erzeugte Wasserstoff teilweise und/oder für eine bestimmte Zeitspanne zur Erzeugung von Biogas, insbesondere zur Methanisierung, eingesetzt wird, wobei der Wasserstoff vorzugsweise in einen separaten Fermenter eingeleitet wird und dort mit CO₂ abreagiert.

Weiterhin ist es bevorzugt, dass der in Schritt d) freigesetzte Wasserstoff teilweise und/oder für eine bestimmte Zeitspanne zur Erzeugung von Biogas, insbesondere zur Methanisierung, eingesetzt wird, wobei der Wasserstoff vorzugsweise in einen separaten Fermenter eingeleitet wird und dort mit CO₂ abreagiert.

Weiterhin ist es bevorzugt, dass der in Schritt c) erzeugte Sauerstoff teilweise und/oder für eine bestimmte Zeitspanne zur Erzeugung von Biogas eingesetzt wird, wobei der Sauerstoff vorzugsweise in einen separaten Fermenter oder Nachgärer eingeleitet wird. Diese Ausführungsform ist deswegen vorteilhaft, weil der bei der Elektrolyse freiwerdende Sauerstoff einem Biogasprozess im Normalbetrieb zugegeben werden kann, um die Qualität des Biogases zu erhöhen. Die Zugabe von Sauerstoff anstelle von Umgebungsluft zur Belüftung des Gärprozesses vermeidet einen erhöhten Stickstoffgehalt im später erhaltenen Biogas.

Weiterhin ist es bevorzugt, dass erzeugtes Biogas, insbesondere Methan, und in den Schritten c) und/oder d) erzeugter oder freigesetzter Wasserstoff miteinander vermischt werden, um sie gemeinsam nach Schritt d) in die Vorrichtung (V2) zu überführen, wo sie in elektrischen Strom und gegebenenfalls in Wärme umgewandelt werden.

Die in den vorgenannten Optionen erwähnten Zeitspannen sind prinzipiell beliebig. Es kann sich dabei um eine oder mehrere Stunden, einen oder mehrere Tage oder sogar um noch längere Zeiträume, wie Wochen und Monate, handeln. Dies hängt ganz vom jeweiligen Bedarf der Erzeugung von Biogas, der Speicherung von Wasserstoff und/oder der Erzeugung von elektrischem Strom ab.

Ein weiterer Gegenstand der vorliegenden Erfindung betrifft ein Verfahren zur Erzeugung von elektrischem Strom und gegebenenfalls von Wärme umfassend die nachfolgenden Schritte a) bis e), wobei das Verfahren in der Reihenfolge der Schritte c), a), b), d) und e) durchgeführt wird:
c) Erzeugung von Wasserstoff in einem Elektrolyseur unter Verwendung von Wasser und elektrischem Strom,
a) Inkontaktbringen eines Trägers (T1) mit dem in Schritt c) erzeugten Wasserstoff zur Speicherung des Wasserstoffs unter Erhalt eines mit Wasserstoff beladenen Trägers (T2),
b) Lagerung des mit Wasserstoff beladenen Trägers (T2) in einer Vorrichtung (V1), die Bestandteil einer Biogasanlage ist oder war,
d) Freisetzung von Wasserstoff aus dem mit Wasserstoff beladenen Träger (T2) im Anschluss an die Lagerung gemäß Schritt b),
e) Überführung des in Schritt d) erhaltenen Wasserstoffs in eine Vorrichtung (V2), wobei in der Vorrichtung (V2) Wasserstoff in elektrischen Strom und gegebenenfalls in Wärme umgewandelt wird.

Die vorstehend beschriebenen Verfahrensschritte a) bis e) im Zusammenhang mit dem Verfahren zur Erzeugung von elektrischem Strom und gegebenenfalls von Wärme werden sinngemäß durchgeführt, entsprechend dem ersten Erfindungsgegenstand, also dem Verfahren zum Speichern und Lagern von Wasserstoff sowie den gegebenenfalls durchgeführten vorstehenden oder nachfolgenden Verfahrensschritten. Dies gilt auch für die entsprechenden bevorzugten, mehr bevorzugten, noch mehr bevorzugten oder besonders bevorzugten Definitionen. Insbesondere wird im Verfahrensschritt d) neben der Freisetzung des Wasserstoffs zusätzlich der in Verfahrensschritt a) eingesetzte Träger (T1) wiedergewonnen.

## Patentansprüche

1. Verfahren zum Speichern und Lagern von Wasserstoff umfassend die nachfolgenden Schritte a) und b):
a) Inkontaktbringen eines Trägers (T1) mit Wasserstoff zur Speicherung des Wasserstoffs unter Erhalt eines mit Wasserstoff beladenen Trägers (T2),
b) Lagerung des mit Wasserstoff beladenen Trägers (T2) in einer Vorrichtung (V1), die Bestandteil einer Biogasanlage ist oder war.

2. Verfahren gemäß Anspruch 1, **dadurch gekennzeichnet, dass** die Vorrichtung (V1) ein Fermenter ist oder durch Umrüstung aus einem Fermenter hergestellt worden ist.

3. Verfahren gemäß Anspruch 1 oder 2, **dadurch gekennzeichnet, dass**
i) in Schritt a) Wasserstoff chemisch oder physisorptiv, vorzugsweise chemisch, an den Träger (T1) gebunden wird, und/oder
ii) der Träger (T1) eine ungesättigte Verbindung, bevorzugt eine Verbindung mit mindestens einer Kohlenstoff-Kohlenstoff-Doppelbindung, mehr bevorzugt eine aromatische oder hetero-aromatische Verbindung, noch mehr bevorzugt ein N-Alkylcarbazol, Toluol, Dibenzyltoluol, Benzyltoluol, Naphthalin oder ein Azaborin, besonders bevorzugt Dibenzyltoluol, ist, und/oder
iii) in Schritt a) der mit Wasserstoff beladene Träger (T2) durch zumindest teilweise oder vollständige Hydrierung, vorzugsweise durch vollständige Hydrierung, mit Wasserstoff aus dem Träger (T1) erhalten wird.

4. Verfahren gemäß einem der Ansprüche 1 bis 3, **dadurch gekennzeichnet, dass** vor Schritt a) ein Schritt c) durchgeführt wird:
c) Erzeugung von Wasserstoff in einer Vorrichtung (V6), vorzugsweise in einem Elektrolyseur, unter Verwendung von Wasser und elektrischem Strom.

5. Verfahren gemäß einem der Ansprüche 1 bis 4, **dadurch gekennzeichnet, dass** nach Schritt b) ein Schritt d) durchgeführt wird:
d) Freisetzung von Wasserstoff aus dem mit Wasserstoff beladenen Träger (T2).

6. Verfahren gemäß Anspruch 5, **dadurch gekennzeichnet, dass** nach Schritt d) ein Schritt e) durchgeführt wird:
e) Überführung des in Schritt d) erhaltenen Wasserstoffs in eine Vorrichtung (V2), wobei in der Vorrichtung (V2) Wasserstoff in elektrischen Strom und gegebenenfalls in Wärme umgewandelt wird.

7. Verfahren gemäß Anspruch 6, **dadurch gekennzeichnet, dass**
i) die Vorrichtung (V2) Bestandteil eines Blockheizkraftwerkes, ein Gasmotor, eine Brennstoffzelle, ein Verbrennungsmotor, ein Verbrennungsmotor mit angeschlossenem Generator, eine Turbine oder eine Wasserstoffturbine mit angeschlossenem Generator, vorzugsweise ein Gasmotor, ist, und/oder
ii) die gegebenenfalls in Schritt e) erzeugte Wärme nach Schritt d) zurückgeleitet wird.

8. Verfahren gemäß einem der Ansprüche 4 bis 7, **dadurch gekennzeichnet, dass**
i) in Schritt c) zusätzlich Sauerstoff erzeugt wird, und/oder
ii) in Schritt c) zusätzlich Sauerstoff erzeugt wird und dieser Sauerstoff in der Vorrichtung (V2) gemäß Schritt e) gemeinsam mit Wasserstoff in elektrischen Strom und gegebenenfalls Wärme umgewandelt wird, wobei der Sauerstoff vor seiner Verwendung in Schritt e) gegebenenfalls zwischengespeichert wird, und/oder
iii) in Schritt c) zusätzlich Sauerstoff erzeugt wird und dieser Sauerstoff zur Optimierung der Haltung von Masttieren in einem landwirtschaftlichen Betrieb, zur Beschleunigung des Pflanzenwachstums in einem landwirtschaftlichen Betrieb oder zur Ozonisierung bei der Trinkwasserreinigung verwendet wird, und/oder
iv) in Schritt c) zusätzlich Wärme frei wird, und die freigewordene Wärme gegebenenfalls in einem landwirtschaftlichen Betrieb verwendet wird oder nach Schritt d) geleitet wird, vorzugsweise wird die in Schritt c) freigesetzte Wärme zwischengespeichert, insbesondere in einem Erdwärmespeicher, bevor sie nach Schritt d) geleitet wird.

9. Verfahren gemäß einem der Ansprüche 1 bis 8, **dadurch gekennzeichnet, dass**
i) in Schritt a) zusätzlich Wärme frei wird, und/oder
ii) in Schritt a) zusätzlich Wärme frei wird und diese freigewordene Wärme in einem landwirtschaftlichen Betrieb verwendet wird, vorzugsweise zur Beheizung von Gewächshäusern, und/oder
iii) in Schritt a) zusätzlich Wärme frei wird und die freigewordene Wärme nach Schritt d) geleitet wird, wobei die Wärme vor ihrer Verwendung in Schritt d) vorzugsweise zwischengespeichert wird, insbesondere in einem Erdwärmespeicher zwischengespeichert wird, und/oder,
iv) Schritt a) in einer separaten Vorrichtung (V3) durchgeführt wird, die vorzugsweise ein chemischer Reaktor ist.

10. Verfahren gemäß einem der Ansprüche 5 bis 9, **dadurch gekennzeichnet, dass**
i) bei der Freisetzung von Wasserstoff gemäß Schritt d) zusätzlich der Träger (T1) wiedergewonnen wird, und/oder
ii) bei der Freisetzung von Wasserstoff gemäß Schritt d) zusätzlich der Träger (T1) wiedergewonnen wird und der Träger (T1) erneut zur Durchführung von Schritt a) verwendet wird, und/oder
iii) bei der Freisetzung von Wasserstoff gemäß Schritt d) zusätzlich der Träger (T1) wiedergewonnen wird und anschließend der Träger (T1) in einer separaten Vorrichtung (V5) oder in der Vorrichtung (V1) gelagert wird, und/oder
iv) Schritt d) in einer separaten Vorrichtung (V4) durchgeführt wird, die vorzugsweise ein chemischer Reaktor ist.

11. Verfahren gemäß einem der Ansprüche 1 bis 10, **dadurch gekennzeichnet, dass**
i) die Vorrichtung (V1) zwei räumlich getrennte Bereiche aufweist, wobei ein erster Bereich zur Lagerung des mit Wasserstoff beladenen Trägers (T2) verwendet wird und ein zweiter Bereich zur Lagerung des Trägers (T1) verwendet wird, und/oder
ii) die Vorrichtung (V1) in ihrem Inneren mit einer Schutzauskleidung versehen wird, bevor Schritt b) und gegebenenfalls die Lagerung des Trägers (T1) durchgeführt wird, und/oder
iii) in die Vorrichtung (V1) eine schwimmende Trennwand eingebaut ist und der mit Wasserstoff beladene Träger (T2) unterhalb der schwimmenden Trennwand gelagert wird, und/oder
iv) die Vorrichtung (V1) Bestandteil einer Biogasanlage war und von allen Substanzen gereinigt war, die sich infolge der Erzeugung von Biogas in ihrem Inneren oder gegebenenfalls in ihren Zuleitungen befunden haben, bevor Schritt b) und gegebenenfalls die Lagerung des Trägers (T1) durchgeführt wird.

12. Verfahren gemäß einem der Ansprüche 1 bis 11, **dadurch gekennzeichnet, dass** das Verfahren in einer Anlage durchgeführt wird, die weiterhin zur Erzeugung von Biogas eingesetzt werden kann.

13. Verfahren gemäß Anspruch 12, **dadurch gekennzeichnet, dass**
i) die Erzeugung von Biogas einerseits und das Speichern und Lagern von Wasserstoff oder die Erzeugung von elektrischem Strom sowie gegebenenfalls von Wärme andererseits parallel auf derselben Anlage durchgeführt werden, und/oder
ii) die Erzeugung von Biogas in einem separaten Fermenter durchgeführt wird, der nicht Bestandteil der Vorrichtung (V1) zur Lagerung des mit Wasserstoff beladenen Trägers (T2) ist.

14. Verfahren gemäß Anspruch 12 oder 13, **dadurch gekennzeichnet, dass**
i) der in Schritt c) erzeugte Wasserstoff teilweise und/oder für eine bestimmte Zeitspanne zur Erzeugung von Biogas, insbesondere zur Methanisierung, eingesetzt wird, wobei der Wasserstoff vorzugsweise in einen separaten Fermenter eingeleitet wird und dort mit CO₂ abreagiert, und/oder
ii) der in Schritt d) freigesetzte Wasserstoff teilweise und/oder für eine bestimmte Zeitspanne zur Erzeugung von Biogas, insbesondere zur Methanisierung, eingesetzt wird, wobei der Wasserstoff vorzugsweise in einen separaten Fermenter eingeleitet wird und dort mit CO₂ abreagiert, und/oder
iii) der in Schritt c) erzeugte Sauerstoff teilweise und/oder für eine bestimmte Zeitspanne zur Erzeugung von Biogas eingesetzt wird, wobei der Sauerstoff vorzugsweise in einen separaten Fermenter oder Nachgärer eingeleitet wird, und/oder
iv) erzeugtes Biogas, insbesondere Methan, und in den Schritten c) und/oder d) erzeugter oder freigesetzter Wasserstoff miteinander vermischt werden, um sie gemeinsam nach Schritt d) in die Vorrichtung (V2) zu überführen, wo sie in elektrischen Strom und gegebenenfalls in Wärme umgewandelt werden.

15. Verfahren zur Erzeugung von elektrischem Strom und gegebenenfalls von Wärme umfassend die nachfolgenden Schritte a) bis e), wobei das Verfahren in der Reihenfolge der Schritte c), a), b), d) und e) durchgeführt wird:
c) Erzeugung von Wasserstoff in einem Elektrolyseur unter Verwendung von Wasser und elektrischem Strom,
a) Inkontaktbringen eines Trägers (T1) mit dem in Schritt c) erzeugten Wasserstoff zur Speicherung des Wasserstoffs unter Erhalt eines mit Wasserstoff beladenen Trägers (T2),
b) Lagerung des mit Wasserstoff beladenen Trägers (T2) in einer Vorrichtung (V1), die Bestandteil einer Biogasanlage ist oder war,
d) Freisetzung von Wasserstoff aus dem mit Wasserstoff beladenen Träger (T2) im Anschluss an die Lagerung gemäß Schritt b),
e) Überführung des in Schritt d) erhaltenen Wasserstoffs in eine Vorrichtung (V2), wobei in der Vorrichtung (V2) Wasserstoff in elektrischen Strom und gegebenenfalls in Wärme umgewandelt wird.
